# EUROPÄISCHE PATENTSCHRIFT

(11) **EP 3 203 978 B1**
(45) Veröffentlichungstag und Bekanntmachung des Hinweises auf die Patenterteilung: **20.11.2019**
(21) Anmeldenummer: 15774556.3
(22) Anmeldetag: 25.09.2015
(51) Int. Cl.: A61K 8/39, A61K 8/04, A61K 8/06, B65D 83/38, B65D 83/62, A61K 8/37, A61Q 19/00, A61K 8/81, B65D 83/14

(54) **KOSMETISCHES SPRAY**
COSMETIC SPRAY
SPRAY COSMETIQUE

(30) Priorität: 09.10.2014 DE 102014220449
(43) Veröffentlichungstag der Anmeldung: 16.08.2017
(73) Patentinhaber: Beiersdorf AG, 20253 Hamburg (DE)
(72) Erfinder: SKUBSCH, Kerstin, 25497 Prisdorf (DE); LÜTTIG, Kaja, 20253 Hamburg (DE); MÜLLER, Claudia, 25499 Tangstedt (DE); WERNER, Regine, 29553 Bienenbüttel (DE); HÜLSHOFF, Anke, 22889 Tangstedt (DE)
(86) Internationale Anmeldenummer: PCT/EP2015/072079
(87) Internationale Veröffentlichungsnummer: WO 2016/055287

(56) Entgegenhaltungen:
- EP-A1- 2 636 401
- WO-A2-2012/167905
- DE-A1-102011 077 037
- FR-A1- 2 924 020
- FR-A1- 2 929 845
- US-A1- 2005 124 705
- Anonymous: "GNPD - 48H Anti-Frizz Milky Serum", , 1. September 2014 (2014-09-01), XP055222789, Gefunden im Internet: URL:http://www.gnpd.com/sinatra/recordpage /2661649/from_search/jCDXUQvfu9/ [gefunden am 2015-10-21]
- Anonymous: "GNPD - Intensive Serum", , 1. August 2014 (2014-08-01), XP055222790, Gefunden im Internet: URL:http://www.gnpd.com/sinatra/recordpage /2609761/from_search/jCDXUQvfu9/ [gefunden am 2015-10-21]
- Anonymous: "GNPD - Emulsion", , 1. Juli 2014 (2014-07-01), XP055222791, Gefunden im Internet: URL:http://www.gnpd.com/sinatra/recordpage /2556917/from_search/jCDXUQvfu9/ [gefunden am 2015-10-21]
- Anonymous: "Technology| BoV | Bag on Valve system | Aerosols filling | Pump Spray | Bag On Valve", , 9. September 2013 (2013-09-09), XP055221968, Gefunden im Internet: URL:https://web.archive.org/web/2013090901 3727/http://www.bagonvalve.com/technology/ [gefunden am 2015-10-19]

## Beschreibung

Die vorliegende Erfindung betrifft ein kosmetisches Spray bestehend aus einer Öl-in-Wasser-Emulsion (O/W-Emulsion) enthaltend Polyglyceryl-10 Stearat sowie einem Sprühapplikator-System.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Die Vielzahl an kommerziell erhältlichen kosmetischen Emulsionen darf jedoch nicht darüber hinwegtäuschen, dass diese Zubereitungen des Standes der Technik eine Reihe von Nachteilen aufweisen.

Insbesondere wenn es sich um Emulsionen handelt, die mit einem Sprühapplikatorsystem z.B. einer Aerosoldose oder einem Bag-on-Valve-System (siehe unten) aus einem Vorratsbehältnis unter Druck direkt auf die Haut aufgetragen werden, tritt das Problem auf, dass die Zubereitungen einerseits Temperatur- und lagerstabil sein sollen und nicht zur vorzeitigen Phasentrennung neigen und andererseits hinreichend dünnflüssig sein müssen, um überhaupt versprühbar zu sein.

Ferner tritt bei diesen durch Überdruck freigesetzten Zubereitungen immer das Problem auf, dass mit zunehmender Anwendungsdauer bzw. bei wiederholten Anwendungen der Druck, mit dem die Zubereitung aus dem Vorratsbehältnis heraus befördert wird, absinkt, so dass die Zubereitung mit abnehmendem Druck durch den Dispenser im Sprühkopf "geschossen" (heraus befördert) wird. Dies führt bei den Zubereitungen des Standes der Technik zu einem im Verlauf der Lebensdauer des Produktes sich stark änderdnen Sprühbild der versprühten Zubereitung. Die Tröpfchenverteilung der versprühten Zubereitung verändert sich zunehmend, die Größe der besprühten Fläche wird (bei konstantem Abstand) größer, das Sprühbild viel ungleichmäßiger und Anzahl der Tröpfchen, die eine zu geringe Beschleunigung erzielen und daher vor Erreichen eines sich in horizontaler Entfernung befindlichen Zieles zu Boden fallen, nimmt zu.

Es war daher die Aufgabe der vorliegenden Erfindung, die Mängel des Standes der Technik zu beseitigen und ein kosmetisches Spray zu entwickeln, dessen Sprühbild über verschiedene Sprühdruckstufen hinweg möglichst konstant bleibt.

Überraschend gelöst wird die Aufgabe durch ein kosmetisches Spray gemäß Anspruch 1.

Zwar kennt der Stand der Technik die DE 102011077017, DE 102011077018, DE 102011077028, DE 102011077031, DE 102011077060, doch konnten diese Schriften nicht den Weg zur vorliegenden Erfindung weisen. Ferner kennt der Stand der Technik die GNPD Mintel Datenbank-Einträge 2661649, 2608761, 2556917, die US 2005/124705, DE 102011077037, FR 2924020, FR 2929845, WO 2012/167905, EP 2636401 sowie "Technoloy/BoV/Bag on Valve System/aerosols filling/Pump Sptray/Bag On Valve", doch konnten diese Schriften nicht den Weg zur vorliegenden Erfindung weisen.

Durch den Überdruck im Druckgasbehälter wird bei diesen Bag-on-Valve-Systemen beim Öffnen des Sprühkopfes der Inhalt des Beutels (hier also die O/W-Emulsion) durch den Sprühkopf nach außen gedrückt und durch den im Sprühkopf vorhandenen Dispenser in kleine Tröpfchen ("Sprühnebel") geteilt. Der Druckausgleich findet also nicht durch das direkte Entweichen des Druckgases aus dem Überdruckgefäß statt, sondern durch das Entleeren des Inhaltes aus dem Vorratsbeutel.

Es ist erfindungsgemäß vorteilhaft, wenn der Überdruck in dem Druckgasbehälter des Bag-on-valve-Applikator-Systems von 2*10⁵ bis 12*10⁵ Pa (2 bis 12 bar) (bezogen auf den Umgebungsdruck von 1,013 bar) beträgt.

Es ist erfindungsgemäß vorteilhaft, wenn der Sprühkopf des Sprühapplikators einen gleichmäßigen Sprühstrahl über den gesamten Lebenszeitraum der Dose aufweist. Aus einer Entfernung von 10 cm wird ein Sprühbild von 5 - 6 cm favorisiert. Beim Abfall des Druckes über den gesamten Lebenszeitraum der Dose sollte das Sprühbild nicht über 8 cm hinausgehen. Erfindungsgemäß vorteilhafte Ausführungsformen der vorliegenden Erfindung sind dadurch gekennzeichnet, dass der Beutel, welcher die O/W-Emulsion enthält aus einem Laminat aus PE/adhesive/PA/adhesive/AL/adhesive/PET gebildet wird.

Erfindungsgemäß besonders bevorzugt ist ein Sprühapplikator mit der folgenden Spezifikation:
Abfüllung 8 bar Überdruck mit Stickstoff
Ventil DU 3527 oder DU 2537 von Aptar®
BOV - Cup: Alu gold laquered - Inner gasket: Buna KA 6712 - Body valve: PP - Spring: Inox c302 - Piston: POM - External gasket: Butyl 1,2 mm Foil: PET12/ALU8/OPA15/PP75

Es ist vorteilhaft im Sinne der vorliegenden Erfindung, wenn die erfindungsgemäße O/W-Emulsion von 0,1 bis 2 Gewichts-% Polyglyceryl-10 Stearat, bezogen auf das Gesamtgewicht der Emulsion enthält.

Es ist bevorzugt im Sinne der vorliegenden Erfindung, wenn die erfindungsgemäße O/W-Emulsion von 0,5 bis 1,0 Gewichts-% Polyglyceryl-10 Stearat, bezogen auf das Gesamtgewicht der Emulsion enthält.

Erfindungsgemäß vorteilhafte Ausführungsformen der vorliegenden Erfindung sind dadurch gekennzeichnet, dass die Emulsion Acrylat/C10-30 Alkylacrylat-Crosspolymer enthält.

In einem solchen Falle ist es erfindungsgemäß von Vorteil, wenn die Emulsion von 0,02 bis 0,2 Gewichts-% Acrylat/C10-30 Alkylacrylat-Crosspolymer, bezogen auf das Gesamtgewicht der Emulsion enthält. Erfindungsgemäß bevorzugt ist dabei der Konzentrationsbereich von 0,05 bis 0,15 Gewichts-%, bezogen auf das Gesamtgewicht der Emulsion.

Es ist erfindungsgemäß vorteilhaft, wenn die Emulsion Capryl/Caprinsäure Triglycerid, Isopropylpalmitat und/oder Sheabutter enthält.

Enthält die Emulsion Capryl/Caprinsäure Triglycerid, so ist es erfindungsgemäß vorteilhaft, wenn dieser Stoff in einer Konzentration von 1 bis 7 Gewichts-%, bezogen auf das Gesamtgewicht der Emulsion in dieser vorhanden ist.

Enthält die Emulsion Isopropylpalmitat, so ist es erfindungsgemäß vorteilhaft, wenn dieser Stoff in einer Konzentration von 1 bis 7 Gewichts-%, bezogen auf das Gesamtgewicht der Emulsion in dieser vorhanden ist.

Enthält die Emulsion Sheabutter, so ist es erfindungsgemäß vorteilhaft, wenn dieser Stoff in einer Konzentration von 0,5 bis 3 Gewichts-%, bezogen auf das Gesamtgewicht der Emulsion in dieser vorhanden ist.

Erfindungsgemäß vorteilhafte Ausführungsformen der vorliegenden Erfindung sind auch dadurch gekennzeichnet, dass die Emulsion Dimethicone und/oder Cyclomethicon enthält.

Die Ölphase der erfindungsgemäßen Emulsion kann darüber hinaus noch weitere Öl-, Fett- und Wachskomponenten enthalten, beispielsweise polaren Öle aus der Gruppe der Lecithine oder Verbindungen wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr. Auch Verbindungen wie Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat können eingesetzt werden.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid gewählt werden. Es ist insbesondere vorteilhaft, wenn die Ölphase der erfindungsgemäßen Formulierungen einen Gehalt an C₁₂₋₁₅-Alkylbenzoat aufweist.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß von Vorteil, wenn die erfindungsgemäße Emulsion Ethanol und/oder Glycerin enthält.

Enthält die Emulsion Ethanol, so ist eine Einsatzkonzentration von 0,5 bis 8 Gewichts-%, bezogen auf das Gesamtgewicht der Emulsion erfindungsgemäß vorteilhaft.

Enthält die Emulsion Glycerin, so ist eine Einsatzkonzentration von 1 bis 12 Gewichts-%, bezogen auf das Gesamtgewicht der Emulsion erfindungsgemäß vorteilhaft.

Vorteilhafte Ausführungsformen der vorliegenden Erfindung sind auch dadurch gekennzeichnet, dass die Emulsion einen oder mehrere Wirkstoffe gewählt aus der Gruppe der Verbindungen UV-Filter, Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

Erfindungsgemäß vorteilhafte UV-Filter können beispielsweise, gewählt werden aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhe-xylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist erfindungsgemäß von Vorteil, wenn die Emulsion Ethylhexylglycerin, Propylenglycol, Butylenglycol, 2-Methylpropan-1,3-diol, 1,2-Pentandiol, 1,2-Hexandiol, 1,2-Octandiol, Piroctone Olamin und/oder 1,2-Decandiol enthält.

Erfindungsgemäß vorteilhafte Ausführungsformen der vorliegenden Erfindung sind dadurch gekennzeichnet, dass die Emulsion Phenoxyethanol und/oder Methylparaben enthält. Dabei ist es erfindungsgemäß vorteilhaft, wenn die Emulsion frei ist von Propyl- und Butylparaben.

Erfindungsgemäß vorteilhafte Ausführungsformen sind ferner dadurch gekennzeichnet, dass die Emulsion mindestens 70 Gewichts-% Wasser, bezogen auf das Gesamtgewicht der Emulsion, enthält.

### Vergleichsversuch

Mit dem folgenden Versuch konnte der erfindungsgemäße Effekt beispielhaft belegt werden:

| | Muster A | Muster B |
|---|---|---|
| Polyglyceryl-10 Stearat | 0,7 | |
| Polyglyceryl-3 Methylglucose Distearat | | 0,7 |
| Acrylates/C10-30 Alkyl Acrylate Crosspolymer | 0,1 | 0,1 |
| Caprylic/Capric Triglycerid | 5 | 5 |
| Isopropyl Palmitat | 5 | 5 |
| Butyrospermum Parkii Butter | 1 | 1 |
| Dimethicon | 1 | 1 |
| Glycerin | 7 | 7 |
| Natronlauge Lösung 45%-ig | 0,07 | 0,07 |
| Phenoxyethanol | 0,8 | 0,8 |
| Methylparaben | 0,3 | 0,3 |
| Aqua | 76,03 | 76,03 |
| Alkohol | 3 | 3 |

Abfüllung 8*10⁵ Pa (8 bar) Überdruck mit Stickstoff Ventil DU 3527 von Aptar® BOV - Cup: Alu gold laquered - Inner gasket: Buna KA 6712 - Body valve: PP - Spring: Inox c302 - Piston: POM - External gasket: Butyl 1,2 mm Foil: PET12/ALU8/OPA15/PP75
Die Muster wurden im Abstand von 10 cm senkrecht auf blaues Papier gesprüht.

Muster A zeigt über die gesamte Anwendungsdauer und damit verbundenen sinkenden Druck (von 8 bar bis zu 3 bar) in der Dose ein relativ gleichmäßiges, rundes Sprühbild von ca. 5-10 cm Durchmesser.

Muster B hingegen verändert mit abnehmendem Druck sein Sprühbild sehr deutlich. Bei voller Dosenfüllung bei ca 8 bar zeigt es einen runden Kreis von ca 8 cm Durchmesser. Mit abnehmender Menge/Druck entwickelt sich das Sprühbild zu einem länglichen zweistrahligen Muster von ca 20 cm Breite. Dadurch kann man das Produkt nicht gleichmäßig auftragen.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | A | B | C | D |
|---|---|---|---|---|
| | % | % | % | % |
| Polyglyceryl-10 Stearate | 0,7 | 0,5 | 0,7 | 0,6 |
| Acrylates/C10-30 Alkyl Acrylate Crosspolymer | 0,12 | 0,1 | 0,1 | 0,1 |
| Xanthan Gummi | | 0,05 | | 0,1 |
| Caprylic/Capric Triglycerid | 5 | 4 | 4 | 2 |
| Isopropyl Palmitat | 5 | | 6 | 3 |
| Butyrospermum Parkii Butter | 1 | | 0,5 | 1 |
| Dimethicon | 0,9 | | 1 | |
| C12-15 Alkylbenzoat | | | | 3 |
| Mandelöl | 2 | | | |
| Dicaprylyl Ether | | 5 | | |
| Glycerin | 7 | 9 | 8 | 5 |
| Phenoxyethanol | 0,8 | 0,5 | 0,5 | 0,5 |
| Methylparaben | 0,3 | 0,2 | 0,3 | 0,3 |
| Kakaobutter | | 1 | | |
| Tocopherolacetat | | 0,5 | | |
| Alkohol | 3 | 1 | 2 | 3 |
| Parfüm | 0,3 | 0,35 | 0,3 | |
| Natronlauge | pH Wert Einstellung | pH Wert Einstellung | pH Wert Einstellung | pH Wert Einstellung |
| Wasser | ad. 100 | ad. 100 | ad. 100 | ad. 100 |
| Anfangsdruck bar | 8 | 9 | 7 | 8 |
| Druckgas | Stickstoff | Stickstoff | Stickstoff | Stickstoff |
| Sprühventil | DU 3527 | DU 3520 | DU 3527 | DU 3527 |

BOV Applikator System z.B. von der Firma Aptar®
Ex-EP BOV - Cup: Alu gold laquered - Inner gasket: Buna KA 6712 - Body valve: PP - Spring: Inox 302 - Piston: POM - External gasket: Butyl 1,2 mm Foil: PET12/ALU8/OPA15/PP75

## Patentansprüche

1. Kosmetisches Spray bestehend aus
a) einer Öl-in-Wasser-Emulsion (O/W-Emulsion) enthaltend Polyglyceryl-10 Stearat sowie
b) einem Sprühapplikator-System, wobei als Sprühapplikator-System ein Bag-on-valve Applikator-System eingesetzt wird, bei dem sich in einem Druckgasbehälter mit Überdruck ein Beutel enthaltend die O/W-Emulsion befindet.

2. Kosmetisches Spray nach Anspruch 1, **dadurch gekennzeichnet, dass** der Überdruck in dem Überdruckbehälter des Bag-on-valve-Applikators von 2*10⁵ bis 12* 10⁵ Pa (2 bis 12 bar) beträgt.

3. Kosmetisches Spray nach einem der vorhergehenden Ansprüche, **dadurch gekennzeichnet, dass** der Beutel, welcher die O/W-Emulsion enthält aus einem Laminat aus PE/adhesive/PA/adhesive/AL/adhesive/PET gebildet wird.

4. Kosmetisches Spray nach einem der vorhergehenden Ansprüche, **dadurch gekennzeichnet, dass** als Sprühapplikatorsystem ein System mit der folgenden Spezifikation verwendet wird: Abfüllung 8 bar Überdruck mit Stickstoff Ventil DU 3527 oder DU 2537 von Aptar® BOV - Cup: Alu gold laquered - Inner gasket: Buna KA 6712 - Body valve: PP - Spring: Inox c302 - Piston: POM - External gasket: Butyl 1,2 mm Foil: PET12/ALU8/OPA15/PP75.

5. Kosmetisches Spray nach einem der vorhergehenden Ansprüche, **dadurch gekennzeichnet, dass** die O/W-Emulsion von 0,1 bis 2 Gewichts-% Polyglyceryl-10 Stearat, bezogen auf das Gesamtgewicht der Emulsion enthält.

6. Kosmetisches Spray nach einem der vorhergehenden Ansprüche, **dadurch gekennzeichnet, dass** die Emulsion Acrylat/C10-30 Alkylacrylat-Crosspolymer enthält.

7. Kosmetisches Spray nach einem der vorhergehenden Ansprüche, **dadurch gekennzeichnet, dass** die Emulsion von 0,02 bis 0,2 Gewichts-% Acrylat/C10-30 Alkylacrylat-Crosspolymer, bezogen auf das Gesamtgewicht der Emulsion enthält.

8. Kosmetisches Spray nach einem der vorhergehenden Ansprüche, **dadurch gekennzeichnet, dass** die Emulsion Capryl/Caprinsäure Triglycerid, Isopropylpalmitat und/oder Sheabutter enthält.

9. Kosmetisches Spray nach einem der vorhergehenden Ansprüche, **dadurch gekennzeichnet, dass** die Emulsion Dimethicone und/oder Cyclomethicon enthält. Kosmetisches Spray nach einem der vorhergehenden Ansprüche, **dadurch gekennzeichnet, dass** die Emulsion Ethanol und/oder Glycerin enthält.

10. Kosmetisches Spray nach einem der vorhergehenden Ansprüche, **dadurch gekennzeichnet, dass** die Emulsion einen oder mehrere Wirkstoffe gewählt aus der Gruppe der Verbindungen UV-Filter, Magnolienextrakt, Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

11. Kosmetisches Spray nach einem der vorhergehenden Ansprüche, **dadurch gekennzeichnet, dass** die Emulsion Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

12. Kosmetisches Spray nach einem der vorhergehenden Ansprüche, **dadurch gekennzeichnet, dass** die Emulsion Phenoxyethanol und/oder Methylparaben enthält.

13. Kosmetisches Spray nach einem der vorhergehenden Ansprüche, **dadurch gekennzeichnet, dass** die Emulsion mindestens 70 Gewichts-% Wasser, bezogen auf das Gesamtgewicht der Emulsion, enthält.

## Claims

1. Cosmetic spray consisting of
a) an oil-in-water emulsion (O/W emulsion) comprising polyglyceryl-10 stearate and also
b) a spray applicator system, wherein the spray applicator system used is a bag-on-valve applicator system, in which a bag containing the O/W emulsion is in a pressurized gas container under positive pressure.

2. Cosmetic spray according to Claim 1, **characterized in that** the positive pressure in the positive pressure container of the bag-on-valve applicator is from 2*10⁵to 12*10⁵ Pa (2 to 12 bar).

3. Cosmetic spray according to either of the preceding claims, **characterized in that** the bag containing the O/W emulsion is formed from a laminate of PE/adhesive/PA/adhesive/AL/adhesive/PET.

4. Cosmetic spray according to any of the preceding claims, **characterized in that** the spray applicator system used is a system having the following specification: filling 8 bar positive pressure with nitrogen valve DU 3527 or DU 2537 from Aptar®, BOV - cup: Alu gold lacquered - inner gasket: Buna KA 6712 - body valve: PP - spring: Inox c302 - piston: POM - external gasket: butyl 1.2 mm foil: PET12/ALU8/OPA15/PP75.

5. Cosmetic spray according to any of the preceding claims, **characterized in that** the O/W emulsion comprises from 0.1 to 2% by weight polyglyceryl-10 stearate, based on the total weight of the emulsion.

6. Cosmetic spray according to any of the preceding claims, **characterized in that** the emulsion comprises acrylate/C10-30 alkyl acrylate crosspolymer.

7. Cosmetic spray according to any of the preceding claims, **characterized in that** the emulsion comprises from 0.02 to 0.2% by weight acrylate/C10-30 alkyl acrylate crosspolymer, based on the total weight of the emulsion.

8. Cosmetic spray according to any of the preceding claims, **characterized in that** the emulsion comprises caprylic/capric triglyceride, isopropyl palmitate and/or shea butter.

9. Cosmetic spray according to any of the preceding claims, **characterized in that** the emulsion comprises dimethicone and/or cyclomethicone.
Cosmetic spray according to any of the preceding claims, **characterized in that** the emulsion comprises ethanol and/or glycerol.

10. Cosmetic spray according to any of the preceding claims, **characterized in that** the emulsion comprises one or more active ingredients selected from the group of compounds comprising UV filters, magnolia extract, glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, 3-alanine and/or licochalcone A.

11. Cosmetic spray according to any of the preceding claims, **characterized in that** the emulsion comprises ethylhexyl glycerin, propylene glycol, butylene glycol, 2-methylpropane-1,3-diol, 1,2-pentanediol, 1,2-hexanediol, 1,2-octanediol and/or 1,2-decanediol.

12. Cosmetic spray according to any of the preceding claims, **characterized in that** the emulsion comprises phenoxyethanol and/or methylparaben.

13. Cosmetic spray according to any of the preceding claims, **characterized in that** the emulsion comprises at least 70% by weight water, based on the total weight of the emulsion.

## Revendications

1. Pulvérisation cosmétique constituée par :
a) une émulsion huile dans eau (émulsion H/E) contenant du stéarate de polyglycéryle-10, et
b) un système d'application de pulvérisation, un système d'application valve à poche (Bag On Valve) étant utilisé en tant que système d'application de pulvérisation, dans lequel une poche contenant l'émulsion H/E se trouve dans un contenant de gaz comprimé présentant une surpression.

2. Pulvérisation cosmétique selon la revendication 1, **caractérisée en ce que** la surpression dans le contenant présentant une surpression de l'applicateur valve à poche est de 2*10⁵ à 12*10⁵ Pa (2 à 12 bar).

3. Pulvérisation cosmétique selon l'une quelconque des revendications précédentes, **caractérisée en ce que** la poche qui contient l'émulsion H/E est formée en un stratifié de PE/adhésif/PA/adhésif/AL/adhésif/PET.

4. Pulvérisation cosmétique selon l'une quelconque des revendications précédentes, **caractérisée en ce qu'**un système présentant la spécification suivante est utilisé en tant que système d'application de pulvérisation : remplissage 8 bar de surpression avec de l'azote, valve DU 3527 ou DU 2537 d'Aptar® BOV - coupelle : alu laqué or - joint intérieur : Buna KA 6712 - valve de corps : PP - ressort : inox c302 - piston : POM - joint extérieur : butyle 1,2 mm - film : PET12/ALU8/OPA15/PP75.

5. Pulvérisation cosmétique selon l'une quelconque des revendications précédentes, **caractérisée en ce que** l'émulsion H/E contient de 0,1 à 2 % en poids de stéarate de polyglycéryle-10, par rapport au poids total de l'émulsion.

6. Pulvérisation cosmétique selon l'une quelconque des revendications précédentes, **caractérisée en ce que** l'émulsion contient Acrylat/C10-30 Alkylacrylat-Crosspolymer.

7. Pulvérisation cosmétique selon l'une quelconque des revendications précédentes, **caractérisée en ce que** l'émulsion contient de 0,02 à 0,2 % en poids d'Acrylat/C10-30 Alkylacrylat-Crosspolymer, par rapport au poids total de l'émulsion.

8. Pulvérisation cosmétique selon l'une quelconque des revendications précédentes, **caractérisée en ce que** l'émulsion contient du triglycéride de l'acide caprylique/caprique, du palmitate d'isopropyle et/ou du beurre de karité.

9. Pulvérisation cosmétique selon l'une quelconque des revendications précédentes, **caractérisée en ce que** l'émulsion contient de la diméthicone et/ou de la cyclométhicone.
Pulvérisation cosmétique selon l'une quelconque des revendications précédentes, **caractérisée en ce que** l'émulsion contient de l'éthanol et/ou de la glycérine.

10. Pulvérisation cosmétique selon l'une quelconque des revendications précédentes, **caractérisée en ce que** l'émulsion contient un ou plusieurs agents actifs choisis dans le groupe constitué par les composés suivants : les filtres UV, l'extrait de magnolia, l'acide glycyrrhizique, l'urée, l'arctiine, l'acide alpha-liponique, l'acide folique, le phytoène, la D-biotine, la coenzyme Q10, l'alpha-glucosylrutine, la carnitine, la carnosine, la caféine, les isoflavonoïdes naturels et/ou synthétiques, le glycérylglucose, la créatine, la créatinine, la taurine, le tocophérol, l'acétate de tocophérol, la β-alanine et/ou la licochalcone A.

11. Pulvérisation cosmétique selon l'une quelconque des revendications précédentes, **caractérisée en ce que** l'émulsion contient de l'éthylhexylglycérine, du propylène glycol, du butylène glycol, du 2-méthylpropane-1,3-diol, du 1,2-pentanediol, du 1,2-hexanediol, du 1,2-octanediol et/ou du 1,2-décanediol.

12. Pulvérisation cosmétique selon l'une quelconque des revendications précédentes, **caractérisée en ce que** l'émulsion contient du phénoxyéthanol et/ou du méthylparabène.

13. Pulvérisation cosmétique selon l'une quelconque des revendications précédentes, **caractérisée en ce que** l'émulsion contient au moins 70 % en poids d'eau, par rapport au poids total de l'émulsion.
